# EUROPÄISCHE PATENTSCHRIFT

(11) **EP 1 116 475 B1**
(45) Veröffentlichungstag und Bekanntmachung des Hinweises auf die Patenterteilung: **16.04.2008**
(21) Anmeldenummer: 01200030.3
(22) Anmeldetag: 08.01.2001
(51) Int. Cl.: A61B 6/03

(54) **Computertomographie-Verfahren zur Erzeugung eines Scannogramms**
Method for generating a scanogram by computed tomography
Procédé de création d'un scanogramme par tomodensiométrie

(30) Priorität: 15.01.2000 DE 10001492
(43) Veröffentlichungstag der Anmeldung: 18.07.2001
(73) Patentinhaber: Philips Intellectual Property & Standards GmbH, 20099 Hamburg (DE); Koninklijke Philips Electronics N.V., 5621 BA Eindhoven (NL)
(72) Erfinder: Proksa, Roland, 52064 Aachen (DE)
(74) Vertreter: Volmer, Georg

(56) Entgegenhaltungen:
- WO-A-99/00054
- DE-C- 4 103 588
- JP-A- 5 317 305
- US-A- 5 612 985
- US-A- 5 960 056
- SHINJI YAMAMOTO ET AL: "IMAGE PROCESSING FOR COMPUTER-AIDED DIAGNOSIS OF LUNG CANCER BY CT (LSCT)" SYSTEMS & COMPUTERS IN JAPAN,US,SCRIPTA TECHNICA JOURNALS. NEW YORK, Bd. 25, Nr. 2, 1. Februar 1994 (1994-02-01), Seiten 67-80, XP000474632 ISSN: 0882-1666

## Beschreibung

Die Erfindung betrifft ein Computertomographie-Verfahren zur Erzeugung eines Scannogramms für einen Computertomographen, der zur Akquisition von Messwerten eine um eine Rotationsachse drehbare Abtasteinheit mit einer Strahlenquelle und einer Detektoreinheit aufweist, wobei während einer Akquisition der Messwerte eine einen Vorschub in Richtung parallel zur Rotationsachse umfassende Relativbewegung zwischen der Abtasteinheit und einem Untersuchungsbereich erfolgt und anschließend eine Ableitung eines Scannogramms aus den Messwerten. Außerdem bezieht sich die Erfindung auf einen Computertomographen, mit dem dieses Verfahren durchgeführt werden kann, und auf ein Computerprogramm, mit dem ein Computertomograph entsprechend gesteuert werden kann.

Die DE 41 03 588 C1 und die JP-A-5 317 305 beschreiben einen Computertomographen, bei dem ein Patient spiralförmig abgetastet wird, wobei aus verschiedenen Projektionsrichtungen Projektionsdaten erfasst werden. Ein Rechner des Computertomographen berechnet aus den Daten ein Schattenbild simultan mit der Aufnahme.

Shinji Yamamoto et al. offenbaren in dem Artikel "Image processing for computer-aided diagnosis of lung cancer by CT (LSCT)", Systems and Computer in Japan, Bd. 25, Nr. 2, 1, Februar 1994, Seiten 67-80 einen Computertomographen, bei dem sich eine Strahlenquelle während der Akquisition helixförmig um einen Patienten bewegt, während Daten akquiriert werden. Aus den Daten werden mehrere Schichtbilder rekonstruiert, die mittels einer sogenannten "maximum intensity projection" visualisiert werden.

Ein Scannogramm von einem Untersuchungsbereich wird bekanntlich dadurch erzeugt, dass zwischen dem Untersuchungsbereich und der Abtasteinheit während der Akquisition der Messwerte eine Relativbewegung erfolgt, die lediglich eine Translation in Richtung parallel zur Rotationsachse umfasst. Die Messwerte gehören dabei zu linien- bzw. streifenförmigen Abschnitten des Untersuchungsbereiches, die zu einem zweidimensionalen Bild - dem Scannogramm - kombiniert werden. Der Rechenaufwand für ein solches Scannogramm ist - verglichen mit der Rekonstruktion eines Computertomogramms - gering. Die Dosis, die erforderlich ist, um ein Scannogramm mit ausreichender Bildqualität zu erzeugen, ist wesentlich geringer als die Dosis, die erforderlich wäre, um mit den üblichen CT-Verfahren (CT = Computertomographie) die Schwächung in einem Abschnitt mit der gleichen Länge zu rekonstruieren.Deshalb werden Scannogramme üblicherweise dazu benutzt, den - im Vergleich zum Scannogramm verkürzten - Bereich festzulegen, der für die Diagnose relevant ist und mit Hilfe eines CT-Verfahrens dargestellt werden soll.

Die üblichen Scannogramme sind jedoch insofern beschränkt, als die Richtung, aus der der Untersuchungsbereich durchstrahlt wird, ebenso vorgegeben ist, wie die durch den Abstand zwischen der Strahlenquelle und der Detektoreinheit bedingte Projektionsgeometrie. Bei der Erstellung eines Scannogramms kann es daher vorkommen, dass der für die Diagnose wichtige Bereich in dem Scannogramm nicht erkannt werden kann, weil er z.B. durch eine Knochenstruktur verdeckt ist. Aufgabe der vorliegenden Erfindung ist es, ein wesentlich flexibleres Verfahren zur Erzeugung eines Scannogramms anzugeben.

Diese Aufgabe wird durch ein Computertomographie-Verfahren gemäß Anspruch 1 gelöst.

Auf den ersten Blick erscheint es paradox, die Messwerte für ein Scannogramm dadurch zu gewinnen, dass - genauso wie bei der eigentlichen CT-Untersuchung - die Abtasteinheit während der Datenakquisition um die Rotationsachse rotiert und die Schwächung in dem Untersuchungsbereich durch ein vergleichsweise aufwendiges Rekonstruktionsverfahren rekonstruiert wird, so dass sich ein 3D-Datensatz ergibt. Diese Schritte werden ja auch bei der eigentlichen CT-Untersuchung durchgeführt, so dass sich die Frage aufdrängt, weshalb man die für die Diagnose relevante Regionen (ROI) für das nachfolgende CT-Verfahren anhand eines Scannogramms festlegen soll, zu dessen Erstellung ebenfalls ein CT-Verfahren durchgeführt werden muß - und zwar in der Regel über einen ausgedehnteren Bereich. Es kommt noch hinzu, dass aus diesem 3D-Datensatz noch ein synthetisches Projektionsbild abgeleitet werden muss, das als Scannogramm dient. Als "synthetisch" wird dieses Projektionsbild deshalb bezeichnet, weil es nicht unmittelbar erzeugt wird wie bei der üblichen Erzeugung eines Scannogramms, sondern aus dem zuvor rekonstruierte 3D-Datensatz berechnet werden muß.

Die Erfindung basiert auf der Erkenntnis, dass man den für die Erzeugung eines Scannogramms benötigten 3D-Datensatz mit einer Dosis akquirieren kann, die nicht größer ist als bei der üblichen Erzeugung eines Scannogramms. Diese Dosis ist wesentlich geringer als die für die eigentliche CT-Untersuchung eines gleich langen Abschnitts benötigte Dosis, und deshalb kann man die Schwächung an den einzelnen Voxeln des Untersuchungsbereichs nur mit einem sehr schlechten Signal/Rauschverhältnis rekonstruieren. Da aber der Bildwert für jedes Pixel in dem Scannogramm aus einer Vielzahl von Voxeln des Untersuchungsbereichs abgeleitet wird, ergibt sich für das Scannogramm, das aus diesem Datensatz abgeleitet wird, ein ausreichendes Signal/Rauschverhältnis. Somit ist die Dosis nicht höher als bei der konventionellen Erzeugung eines Scannogramms.

Dafür ist man bei der Wahl der Projektionsgeometrie völlig frei. Man kann z.B. das Scannogramm aus dem 3D-Datensatz durch eine Parallelprojektion ableiten, bei der auf parallelen Strahlen liegende Voxel zur Berechnung des Bildwertes in dem Pixel herangezogen werden, in dem der betreffende Strahl endet. Es können Projektionsbilder mit frei wählbarer Projektionsrichtung erzeugt werden, und zwar auch mit einer zur Rotationsachse nicht senkrechten (schrägen) Projektionsrichtung. Die Projektionsgeometrie muß erst nach der Akquisition der Meßwerte für den 3D-Datensatz festgelegt werden, und es können aus einem 3D-Datensatz mehrere unterschiedliche Scannogramme abgeleitet werden, ohne dass erneut Meßwerte akquiriert werden müssen und der Patient einer weiteren Strahlenbelastung ausgesetzt wird.

Mit einem CT-Verfahren müssen dünne und dicke Patienten untersucht werden. Wenn man die Untersuchung sämtlicher Patienten jeweils mit der gleichen Strahlenintensität und der gleichen Strahlenqualität durchführen würde, dann würde sich entweder bei einem dünnen Patienten eine zu hohe Strahlenbelastung oder bei einem dicken Patienten ein ungenügendes Signal/Rauschverhältnis ergeben. Mit der Ausgestaltung nach Anspruch 2 ist es möglich, bei einer nachfolgenden CT-Untersuchung Strahlenqualität und/oder Strahlenintensität so vorzugeben, dass sich keine unnötige Strahlenbelastung und ein ausreichendes Signal/Rauschverhältnis ergibt.

Auch wenn man eine solche Anpassung an den jeweiligen Patienten durchgeführt hat, können Probleme daraus erwachsen, dass bei seitlicher (lateraler) Durchstrahlung eine stärkere Schwächung der Strahlung erfolgt als bei einer Durchstrahlung von vorne (a.-p.), so dass entweder die Strahlenbelastung für die eine Durchstrahlungsrichtung zu hoch oder das Signal/Rauschverhältnis für die andere Durchstrahlungsrichtung zu klein wird, wenn man einen Patienten mit konstanter Strahlenintensität und konstanter Strahlenqualität untersucht. Dies läßt sich mit der in Anspruch 3 angegebenen Ausgestaltung der Erfindung vermeiden, die es möglich macht, für sämtliche Durchstrahlungsrichtungen, d.h. für sämtliche Positionen der Abtasteinheit, ein bestimmtes Signal/Rauschverhältnis bei geringstmöglicher Strahlenbelastung zu erreichen.

Grundsätzlich könnte man mit einem solchen Computertomographen die Meßwerte dadurch akquirieren, dass man die Abtasteinheit in einer bestimmten Position rotieren läßt, um die Meßwerte für die Rekonstruktion einer Schicht oder einer Scheibe zu gewinnen, danach die Abtasteinheit in Richtung der Rotationsachse verschiebt, um die Meßwerte für eine benachbarte Schicht oder Scheibe zu gewinnen usw.. Die nach der Weiterbildung gemäß Anspruch 5 erfolgende helixförmige Relativbewegung, bei der kontinuierlich eine Rotation und ein Vorschub erfolgt, ist demgegenüber aber vorteilhafter, weil sich hierbei ein ruckfreier Übergang von einer Strahlenquellenposition in die andere ergibt.

Anspruch 6 beschreibt eine vorteilhafte weitere Ausgestaltung des Computertomographen. Man könnte das Scannogramm zwar auch mit einem Computertomographen erzeugen, bei dem die Strahlenquelle ein fächerförmiges Strahlenbündel (fan beam) emittiert und die Detektoreinheit nur eine einzige Zeile umfaßt, doch erlaubt es die Ausgestaltung nach Anspruch 6, die Meßwerte schneller zu akquirieren.

Anspruch 7 beschreibt die Software für die Steuereinheit des Computertomographen, mit deren Hilfe sich das erfindungsgemäße Verfahren durchführen läßt.

Die Erfindung wird nachstehend anhand der Zeichnungen näher erläutert. Es zeigen
Fig. 1 einen Computertomographen, der das erfindungsgemäße Verfahren durchführen kann,
Fig. 2 ein Ablaufdiagramm eines Untersuchungsverfahrens mit einem solchen Computertomographen und
Fig. 3 die Erzeugung eines synthetischen Projektionsbildes aus einem 3D-Datensatz.

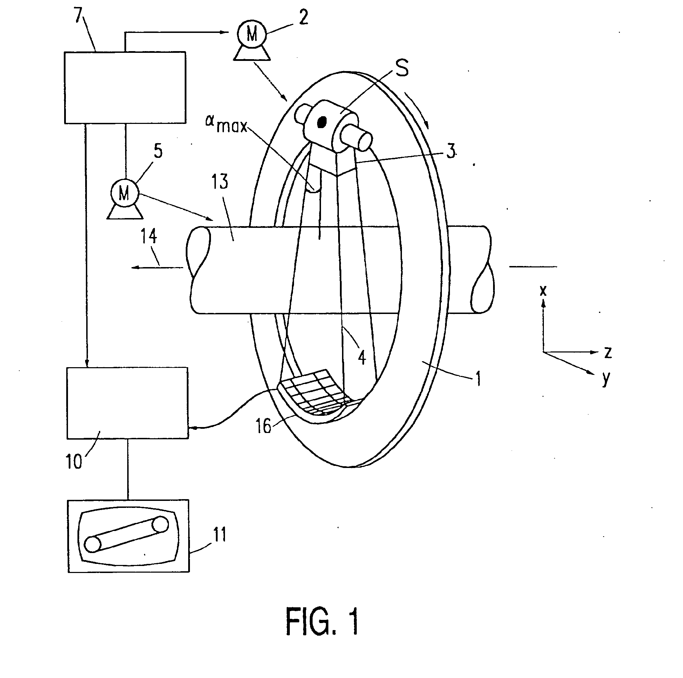

Der in Fig. 1 dargestellte Computertomograph umfaßt eine Abtasteinheit in Form einer Gantry 1, die um eine Rotationsachse 14 rotieren kann. Dazu wird die Gantry von einem Motor 2 mit einer vorzugsweise konstanten, aber einstellbaren Winkelgeschwindigkeit angetrieben. An der Gantry 1 ist eine Strahlenquelle S befestigt, beispielsweise ein Röntgenstrahler. Dieser ist mit einer Kollimatoranordnung 3 versehen, die aus der von der Strahlenquelle S erzeugten Strahlung ein kegelförmiges Strahlenbündel 4 ausblendet, d.h. ein Strahlenbündel, das sowohl in einer zur Rotationsachse senkrechten Ebene als auch in Richtung der Rotationsachse eine von Null verschiedene endliche Ausdehnung hat.

Das Strahlenbündel 4 durchdringt einen Untersuchungsbereich 13, in dem sich ein Patient auf einem Patientenlagerungstisch (beides nicht näher dargestellt) befinden kann. Der Untersuchungsbereich 13 hat die Form eines Zylinders. Nach dem Durchsetzen dieses Zylinders trifft das Röntgenstrahlenbündel 4 auf eine an der Gantry 1 befestigte zweidimensionale Detektoreinheit 16, die eine Vielzahl von matrixförmig angeordneten Detektorelementen umfaßt. Jedes Detektorelement kann in jeder Strahlenquellenposition einen Meßwert für einen Strahl aus dem Strahlenbündel 4 liefern. Die Detektorelemente sind in Zeilen und Spalten angeordnet. Die Detektorspalten verlaufen parallel zur Rotationsachse, die Detektorzeilen können sich in zur Rotationsachse senkrechten Ebenen befinden, beispielsweise auf einem Kreisbogen um die Strahlenquelle S. Die Detektorzeilen enthalten in der Regel wesentlich mehr Detektorelemente (z.B. 1.000) als die Detektorspalten (z.B. 16).

Der mit αₘₐₓ bezeichnete Öffnungswinkel des Strahlenbündels 4 (als Öffnungswinkel ist der Winkel definiert, den ein Strahl, der in einer zur Rotationsachse 14 senkrechten Ebene am Rande des Strahlenbündels 4 liegt, mit einer durch die Strahlenquelle S und die Rotationsachse 14 definierten Ebene einschließt) bestimmt dabei den Durchmesser des Zylinders, innerhalb dessen sich das zu untersuchende Objekt bei der Akquisition der Meßwerte befinden muß. Das Untersuchungsobjekt bzw. der Patientenlagerungstisch kann mittels eines Motors 5 auch parallel zur Rotationsachse 14 verschoben werden. Die Geschwindigkeit dieses Vorschubs ist vorzugsweise konstant und einstellbar. Wenn die Motoren 5 und 2 gleichzeitig laufen, ergibt sich eine helixförmige Abtastbewegung der Strahlenquelle S und der Detektoreinheit 16.

Die von der Detektoreinheit 16 auf der rotierenden Gantry 1 akquirierten Meßdaten werden einem Bildverarbeitungsrechner 10 zugeführt, der sich in der Regel an einem festen Punkt im Raum befindet und mit der Detektoreinheit über einen kontaktlos arbeitenden, nicht näher dargestellten Datenschleifring verbunden ist. Der Bildverarbeitungsrechner 10 kann verschiedene Bildverarbeitungsoperationen durchführen. Aus den akquirierten Meßwerten wird die Schwächung der Röntgenstrahlung in dem Untersuchungsbereich 13 rekonstruiert, so dass sich ein 3D-Datensatz ergibt, und andererseits werden aus diesem 3D-Datensatz synthetische Projektionsbilder abgeleitet, die als Scannogramm dienen.

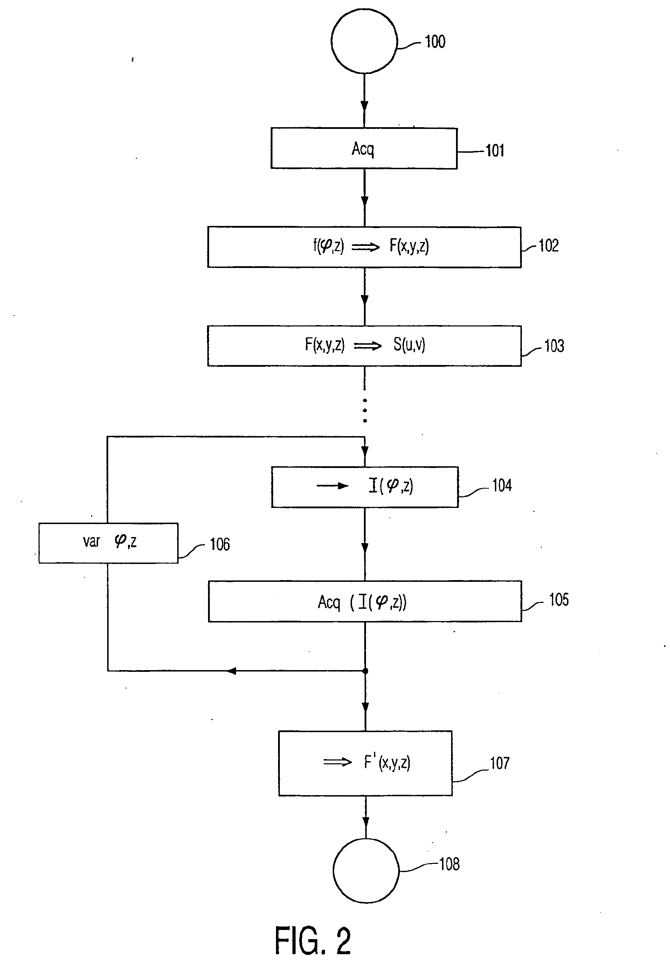

Im folgenden soll anhand des in Fig. 2 dargestellten Ablaufdiagramms das erfindungsgemäße Verfahren erläutert werden. Nach der Initialisierung im Block 100 erfolgt im Block 101 die Akquisition der Meßwerte. Dazu werden die Motoren 2 und 5 eingeschaltet, so dass die Gantry um den Untersuchungsbereich 13 rotiert und die Patientenlagerungsplatte mit dem darauf befindlichen Patienten in Richtung parallel zur Rotationsachse 14, d.h. in z-Richtung des dargestellten kartesischen Koordinatensystems verschoben wird. Gleichzeitig wird die Strahlenquelle eingeschaltet, und zwar mit einer Intensität, die wesentlich geringer ist als bei der späteren CT-Untersuchung. Die Detektoreinheit 16 liefert in jeder Strahlenquellenposition (die durch die Position ϕ der Strahlenquelle S in bezug auf die Rotationsachse 14 bzw. durch die z-Koordinate der Tischplatte definiert ist) einen Satz von Meßwerten f(ϕ, z), die das Integral der Schwächung entlang der einzelnen Strahlen des Strahlenbündels S durch den Untersuchungsbereich darstellen.

Nach dieser Akquisition der Meßwerte folgt im Schritt 102 die Rekonstruktion der Absorptionsverteilung im Untersuchungsbereich. Dabei wird aus den Meßwerten, die das Linienintegral der Schwächung entlang des jeweiligen Strahls darstellen, die Schwächung der Strahlung in den einzelnen, durch ihre Position x,y,z definierten Voxeln des Untersuchungsbereichs 13 rekonstruiert. Es resultiert somit ein 3D-Datensatz F(x,y,z). Ein geeignetes Rekonstruktionsverfahren ist in der WOA-99/36885 beschrieben. Dabei werden für jedes Voxel nur diejenigen Meßwerte akquiriert bzw. zur Rekonstruktion herangezogen, bei deren Akquisition die Strahlenquelle das betreffende Voxel aus einem Winkelbereich von exakt 180° - bezogen auf das jeweilige Voxel - bestrahlt. Das bekannte Verfahren erlaubt es, mit der Rekonstruktion schon zu beginnen, bevor die Akquisition der Meßwerte im Schritt 101 beendet ist.

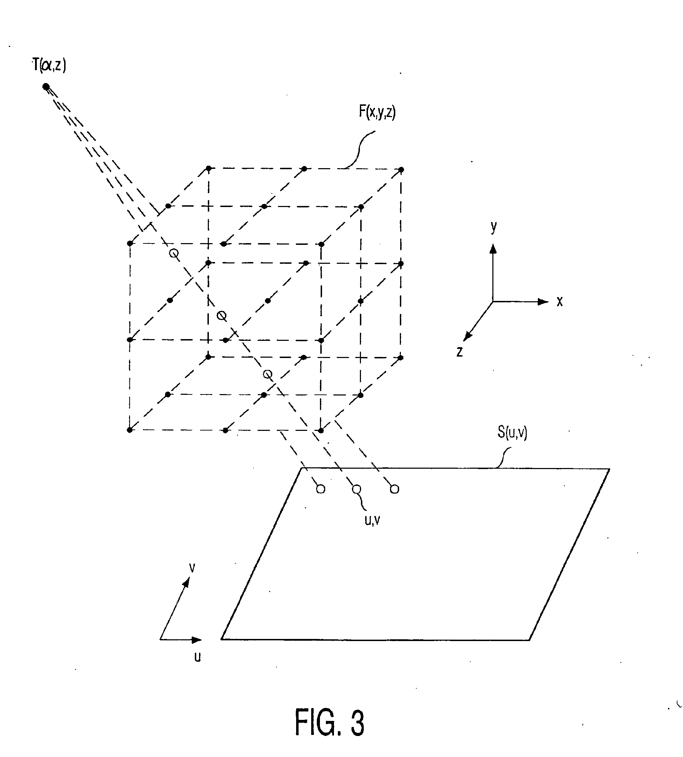

Im nächstfolgenden Schritt 103 wird aus dem im Schritt 102 rekonstruierten 3D-Datensatz F(x,y,z) ein Scannogramm S(u,v) berechnet, d.h. ein zweidimensionales synthetisches Projektionsbild. Dies wird im folgenden anhand der Fig. 3 erläutert. Der Untersuchungsbereich, für den der 3D-Datensatz rekonstruiert ist, ist darin durch einen Kubus mit einem kartesischen Gitter definiert, dessen durch ein "ausgefüllten Punkt" (•) symbolisierte Gitterpunkte die Voxel des Untersuchungsbereichs bzw. das Zentrum dieser Voxel darstellen.

Das Scannogramm wird dadurch erzeugt, dass der rekonstruierte Untersuchungsbereich von einem beliebig wählbaren Projektionsursprung T(α,z) auf eine Fläche projiziert wird. Jedem Pixel u,v in diesem Scannogramm wird dabei ein Bildwert zugeordnet, der von den Schwächungswerten in dem Untersuchungsbereich entlang des Strahles abhängt, der das betreffende Pixel u,v mit dem Projektionsursprung T(α,z) verbindet. Für einen dieser Strahlen sind innerhalb des Untersuchungsbereiches in gleichmäßigen Abständen durch einen "leeren Punkt" (o) symbolisierte Strahlenpunkte angedeutet. Wenn ein Strahlenpunkt mit einem Voxel zusammenfällt, wird ihm der für dieses Voxel ermittelte Schwächungswert zugeordnet. Anderenfalls wird dem Strahlenpunkt ein Schwächungswert zugeordnet, der sich durch eine kubische Interpolation aus den Schwächungswerten der diesem Strahlenpunkt benachbarten Voxel ergibt.

Der Bildwert an dem Pixel, das dem betreffenden Strahl zugeordnet ist, kann beispielsweise durch Summierung der Schwächung an den Strahlenpunkten oder durch eine gewichtete Summierung ermittelt werden. Es kann jedoch auch der Maximalwert der Schwächung entlang des Strahls herangezogen werden oder ein Wert, wie er mit den üblichen sogenannten "Volume-Rendering"-Methoden bestimmt werden kann.

Das Scannogramm wird darüber hinaus auch von der Projektionsgeometrie bestimmt, d.h. von der Lage des Projektionspunktes T(α,z) und des Scannogramms S(u,v) in bezug auf den Untersuchungsbereich. Wenn beispielsweise der Projektionsursprung ins Unendliche versetzt würde, ergäbe sich eine Parallelstrahlgeometrie. Daneben kann auch die Projektionsrichtung α geändert werden, d.h. die Lage des Projektionsursprungs bezogen auf die z-Achse bzw. die Rotationsachse 14. Schließlich ist auch eine Schrägprojektion möglich, wobei ein Strahl vom Projektionsursprung zum Mittelpunkt des Scannogramms mit der Rotationsachse 14 bzw.der z-Achse einen von 90° verschiedenen Winkel einschließt. Aus einem Datensatz können auch verschiedene Scannogramme - auch mit verschiedenen Projektionsgeometrien - berechnet werden.

Nachdem auf diese Weise ein geeignetes Scannogramm erstellt worden ist, kann die eigentliche CT-Untersuchung anhand des Scannogramms geplant werden. Insbesondere kann die Lage und die Länge des Teils des Untersuchungsbereichs festgelegt werden, der bei der eigentlichen CT-Untersuchung dargestellt werden soll. Für diese nachfolgende Untersuchung kann dann - für jede einzelne Position der Strahlenquelle auf der helixförmigen Trajektorie, die diesem im Bezug auf den Untersuchungsbereich 13 beschreibt - im Schritt 104 ein geeigneter Wert für die Intensität und/oder die Qualität der von der Strahlenquelle S erzeugten Strahlung vorausberechnet werden.

Wenn die erwähnte Trajektorie bei der eigentlichen CT-Untersuchung identisch ist mit einem Teil der Trajektorie während der Akquisition der Meßwerte im Schritt 101, ist die Vorausberechnung der Intensität I(ϕ,z) im Schritt 104 relativ einfach. Aus dem Satz von Meßwerten, der in der Position ϕ,z im Schritt 101 akquiriert wurde, wird im Schritt 104 ein Wert für die Intensität I(ϕ,z) so abgeleitet und eingestellt, dass sich ein optimaler Kompromiß zwischen Strahlenbelastung und Bildqualität ergibt. Grundsätzlich ist die eingestellte Intensität um so größer, je stärker die Strahlung in der Position ϕ,z absorbiert bzw. geschwächt wird.
Wenn die Trajektorie im Schritt 101 und bei der nachfolgenden CT-Untersuchung nicht übereinstimmen, ist es aber auch möglich, für jede einzelnen Position anhand des rekonstruierten 3D-Datensatzes F(x,y,z) und der jeweiligen Position ϕ,z der Strahlenquelle die mittlere Schwächung zu berechnen und die Intensität davon abhängig vorzugeben.

Mit der so berechneten und an der Strahlenquelle S eingestellten Strahlenintensität wird im Schritt 105 in der Position ϕ, z ein Satz von Meßwerten akquiriert. Danach erfolgt im Schritt 106 ein Übergang auf eine benachbarte Strahlenquellenposition, und es wird für diese Position im Schritt 104 erneut eine vorausberechnete Intensität eingestellt und bei dieser Einstellung und in dieser Position ein weiterer Satz von Meßwerten akquiriert. Die Schleife 104...106 wird dann so oft durchlaufen, bis für alle Positionen auf der zuvor festgelegten Trajektorie ein Satz von Meßwerten akquiriert worden ist. Danach kann im Schritt 107 eine Rekonstruktion der Absorptionsverteilung erfolgen. Der sich aus dieser Rekonstruktion ergebende 3D-Datensatz F (x,y,z) stellt die Schwächung der Strahlung in dem interessierenden Bereich (ROI) dar. Er hat aber aufgrund der höheren Intensität der Strahlung bei der eigentlichen CT-Untersuchung ein wesentlich besseres Signal/Rauschverhältnis als die Schwächungswerte F(x,y,z), die sich aus der Rekonstruktion der Schwächung für die Erzeugung des Scannogramms ergeben. Danach ist das Verfahren beendet (Block 108).

## Patentansprüche

1. Computertomographie-Verfahren zur Erzeugung eines Scannogramms für einen Computertomographen, der zur Akquisition von Messwerten eine um eine Rotationsachse drehbare Abtasteinheit mit einer Strahlenquelle und einer Detektoreinheit aufweist, wobei zur Erzeugung eines Scannogramms folgende Schritte durchgeführt werden:
a) Ausführung einer Relativbewegung zwischen der Abtasteinheit und einem Untersuchungsbereich, die eine Rotation der Abtasteinheit um die Rotationsachse und einen Vorschub in Richtung parallel zur Rotationsachse umfasst, während der Akquisition der Messwerte,
(b) Rekonstruktion der Absorptionsverteilung im Untersuchungsbereich als aus Voxeln bestehenden 3D-Datensatz, wobei aus den Messwerten, die das Linienintegral der Schwächung entlang des jeweiligen Strahls darstellen, die Schwächung der Strahlung in den einzelnen, durch ihre Position (x, y, z) definierten Voxeln des Untersuchungsbereichs berechnet wird,
c) Berechnung wenigstens eines Scannogramms als synthetisches Projektionsbild aus dem rekonstruierten 3D-Datensatz, wobei der rekonstruierte 3D-Datensatz von einem beliebig wählbaren Projektionsursprung auf eine Fläche projiziert wird.

2. Computertomographie-Verfahren nach Anspruch 1 mit dem weiteren Schrit:
• Steuern der Qualität und/oder der Intensität (I) der Strahlung bei der Akquisition von Messwerten für eine nachfolgende CT-Untersuchung in Abhängigkeit von der Schwächung.

3. Computertomographie-Verfahren nach Anspruch 1 mit den weiteren Schritten:
• Bestimmung der Schwächung der Strahlung in dem Untersuchungsbereich für die einzelnen Positionen (ϕ, z) der Abtasteinheit relativ zum Untersuchungsbereich bei einer nachfolgenden CT-Untersuchung aus dem 3D-Datensatz,
• Steuern der Qualität und/oder der Intensität der (I(ϕ, z)) Strahlung bei der Akquisition von Messwerten in den einzelnen Positionen der nachfolgenden CT-Untersuchung in Abhängigkeit von der Schwächung.

4. Computertomograph zur Durchführung des Verfahrens nach Anspruch 1 mit
• einer um eine Rotationsachse drehbare Abtasteinheit mit einer Strahlenquelle und einer Detektoreinheit zur Akquisition von Messwerten,
• einer Antriebsanordnung für eine zwischen der Abtasteinheit einerseits und dem Untersuchungsbereich andererseits erfolgende Relativbewegung, die eine Rotation um eine Rotationsachse und einen Vorschub parallel zur Rotationsachse umfasst,
• einer Rekonstruktionseinheit zur Rekonstruktion eines von der Schwächung der Strahlung in dem von der Abtasteinheit erfassten Untersuchungsbereich dreidimensional abhängigen 3D-Datensatzes aus den Messwerten und zur weiteren Verarbeitung des 3D-Datensatzes und zur Durchführung weiterer Bildverarbeitungsoperationen, sowie mit
• einer Steuereinheit zur Steuerung der Abtasteinheit, der Antriebsanordnung und der Rekonstruktionseinheit
wobei die Steuereinheit so programmiert ist, dass zur Erzeugung eines Scannogramms die folgenden Schritte ausgeführt werden:
a) Ausführung einer Relativbewegung zwischen der Abtasteinheit und einem Untersuchungsbereich, die eine Rotation der Abtasteinheit um die Rotationsachse und einen Vorschub in Richtung parallel zur Rotationsachse umfasst, während der Akquisition der Messwerte,
(b) Rekonstruktion der Absorptionsverteilung im Untersuchungsbereich als aus Voxeln bestehenden 3D-Datensatz, wobei aus den Messwerten, die das Linienintegral der Schwächung entlang des jeweiligen Strahls darstellen, die Schwächung der Strahlung in den einzelnen, durch ihre Position (x, y, z) definierten Voxeln des Untersuchungsbereichs berechnet wird,
c) Berechnung wenigstens eines Scannogramms als synthetisches Projektionsbild aus dem rekonstruierten 3D-Datensatz, wobei der rekonstruierte 3D-Datensatzvon einem beliebig wählbaren Projektionsursprung auf eine Fläche projiziert wird.

5. Computertomograph nach Anspruch 4,
**dadurch gekennzeichnet,**
**dass** die Steuereinheit so programmiert ist, dass die Rotation und der Vorschub gleichzeitig während Akquisition der Messwerte erfolgen, so dass sich eine helixförmige Relativbewegung ergibt.

6. Computertomograph nach Anspruch 4,
**dadurch gekennzeichnet,**
**dass** das von der Strahlenquelle emittierte Strahlenbündel die Form eines Kegels hat, und dass die das Strahlenbündel erfassende Detektoreinheit mehrere in Richtung parallel zur Rotationsachse gegeneinander versetzte Zeilen von Detektorelementen aufweist.

7. Computerprogramm für eine Steuereinheit zur Steuerung der Abtasteinheit, der Antriebsanordnung und der Rekonstruktionseinheit eines Computertomographen zur Durchführung des Verfahrens nach Anspruch 1 wobei das Computerprogramm zur Erzeugung eines Scannogramms folgende Schritte durchführt:
a) Ausführung einer Relativbewegung zwischen der Abtasteinheit und einem Untersuchungsbereich, die eine Rotation der Abtasteinheit um die Rotationsachse und einen Vorschub in Richtung parallel zur Rotationsachse umfasst, während der Akquisition der Messwerte,
(b) Rekonstruktion der Absorptionsverteilung im Untersuchungsbereich als aus Voxeln bestehenden 3D-Datensatz, wobei aus den Messwerten, die das Linienintegral der Schwächung entlang des jeweiligen Strahls darstellen, die Schwächung der Strahlung in den einzelnen, durch ihre Position (x, y, z) definierten Voxeln des Untersuchungsbereichs berechnet wird,
c) Berechnung wenigstens eines Scannogramms als synthetisches Projektionsbild aus dem rekonstruierten 3D-Datensatz, wobei der rekonstruierte 3D-Datensatzvon einem beliebig wählbaren Projektionsursprung auf eine Fläche projiziert wird.

## Claims

1. A computed tomography method for generating a scannogram for a computed tomography apparatus which, for the acquisition of measured values, includes a scanning unit that is rotatable about an axis of rotation and is provided with a radiation source and a detector unit, with the following steps being carried out for generating a scannogram:
a. performance of a relative motion between the scanning unit and an examination zone, which motion includes a rotation of the scanning unit about the axis of rotation and a displacement in the direction parallel with the axis of rotation during the acquisition of the measured values,
b. reconstruction of the absorption distribution in the examination zone as a 3D data set comprising voxels, from which measured values representing the line integral of the attenuation along the respective ray is computed the attenuation of the radiation in the individual voxels of the xamination zone defined by their position (x, y, z),
c. calculation of at least one scannogram as a synthetic projection image from the reconstructed 3D data set, which reconstructed 3D data set is projected on a plane from a randomly selectable projection origin.

2. A computed tomography method as claimed in claim 1, including the further step of:
• controlling, in dependence on the attenuation, the quality and/or the intensity (I) of the radiation during the acquisition of measured values for a subsequent CT examination.

3. A computed tomography method as claimed in claim 1, which also includes the following steps of:
• determining the attenuation of the radiation in the examination zone for the individual positions (ϕ,z) of the scanning unit relative to the examination zone from the 3D data set during a subsequent CT examination,
• controlling, in dependence on the attenuation, the quality and/or the intensity of the (I(ϕ,z)) radiation during the acquisition of measured values in the individual positions during the subsequent CT examination.

4. A computed tomography apparatus for carrying out the method claimed in claim 1, including
• a scanning unit which is rotatable about an axis of rotation and is provided with a radiation source and a detector unit for the acquisition of measured values,
• a drive device for realizing a relative motion which occurs between the scanning unit on the one side and the examination zone on the other side and includes a rotation about an axis of rotation and a displacement parallel to the axis of rotation,
• a reconstruction unit for reconstructing from the measured values a 3D data set which is three-dimensionally dependent on the attenuation of the radiation in the examination zone covered by the scanning unit, for further processing the 3D data set and for carrying out further image processing operations, as well as
• a control unit for controlling the scanning unit, the drive device and the reconstruction unit,
wherein the control unit is programmed in such a manner that the following steps are carried out for generating a scannogram:
a) performance of a relative motion between the scanning unit and an examination zone, which motion includes a rotation of the scanning unit about the axis of rotation and a displacement in the direction parallel with the axis of rotation during the acquisition of the measured values,
b) reconstruction of the absorption distribution in the examination zone as a 3D data set comprising voxels, from which measured values representing the line integral of the attenuation along the respective ray is computed the attenuation of the radiation in the individual voxels of the xamination zone defined by their position (x, y, z),
c) calculation of at least one scannogram as a synthetic projection image from the reconstructed 3D data set, which reconstructed 3D data set is projected on a plane from a randomly selectable projection origin.

5. A computed tomography apparatus as claimed in claim 4,
**characterized in that** the control unit is programmed in such a manner that the rotation and the displacement take place simultaneously during the acquisition of the measured values, thus resulting in a helical relative motion.

6. A computed tomography apparatus as claimed in claim 4,
**characterized in that** the radiation beam emitted by the radiation source is shaped as a cone and that the detector unit detecting the radiation beam includes a plurality of rows of detector elements which are offset relative to one another in the direction parallel to the axis of rotation.

7. A computer program for a control unit for controlling the scanning unit, the drive device and the reconstruction unit of a computed tomography apparatus for carrying out the method claimed in claim 1:
which computer program carries out the following steps for generating a scannogram:
a) performance of a relative motion between the scanning unit and an examination zone, which motion includes a rotation of the scanning unit about the axis of rotation and a displacement in the direction parallel with the axis of rotation during the acquisition of the measured values,
b) reconstruction of the absorption distribution in the examination zone as a 3D data set comprising voxels, from which measured values representing the line integral of the attenuation along the respective ray is computed the attenuation of the radiation in the individual voxels of the xamination zone defined by their position (x, y, z),
c) calculation of at least one scannogram as a synthetic projection image from the reconstructed 3D data set, which reconstructed 3D data set is projected on a plane from a randomly selectable projection origin.

## Revendications

1. Procédé de tomographie informatisée pour la production d'un scannogramme pour un tomographe informatisé qui présente pour l'acquisition de valeurs de mesure une unité de balayage tournant autour d'un axe de rotation avec une source de rayons et une unité à détecteurs, les étapes suivantes étant exécutées pour la production d'un scannogramme :
a) exécution d'un mouvement relatif entre l'unité de balayage et une zone d'examen qui comprend une rotation de l'unité de balayage autour de l'axe de rotation et une avance dans la direction parallèle à l'axe de rotation pendant l'acquisition des valeurs de mesure;
b) reconstruction de la distribution de l'absorption dans la zone d'examen comme un jeu de données en 3D composé de voxels, l'atténuation du rayonnement dans les différents voxels de la zone d'examen définis par leur position (x, y, z) étant calculée à partir des valeurs de mesure qui représentent l'intégrale de ligne de l'atténuation le long du faisceau respectif;
c) calcul d'au moins un scannogramme comme image de projection synthétique à partir du jeu de données en 3D reconstruit, le jeu de données en 3D reconstruit étant projeté sur une surface à partir d'une origine de projection à choisir librement.

2. Procédé de tomographie informatisée selon la revendication 1 avec une étape supplémentaire:
• commande de la qualité et/ou de l'intensité (I) du rayonnement pour l'acquisition de valeurs de mesure pour un examen CT consécutif en fonction de l'atténuation.

3. Procédé de tomographie informatisée selon la revendication 1 avec les étapes supplémentaires :
• détermination de l'atténuation du rayonnement dans la zone d'examen pour les différentes positions (ϕ, z) de l'unité de balayage par rapport à la zone d'examen lors d'un examen CT consécutif à partir du jeu de données en 3D;
• commande de la qualité et/ou de l'intensité du rayonnement (1(ϕ,z)) lors de l'acquisition de valeurs de mesure dans les différentes positions de l'examen CT suivant en fonction de l'atténuation.

4. Tomographie informatisée de mise en oeuvre du procédé selon la revendication 1 avec
• une unité de balayage tournant autour d'un axe de rotation avec une source de rayons et une unité à détecteurs pour l'acquisition de valeurs de mesure;
• un dispositif d'entraînement pour un mouvement relatif intervenant entre l'unité de balayage, d'une part, et la zone d'examen, d'autre part, qui comprend une rotation autour d'un axe de rotation et une avance parallèlement à l'axe de rotation;
• une unité de reconstruction pour la reconstruction d'un jeu de données en 3D dépendant tridimensionnellement de l'atténuation du rayonnement dans la zone d'examen couverte par l'unité de balayage à partir des valeurs de mesure et pour le traitement ultérieur du jeu de données en 3D et pour l'exécution d'opérations supplémentaires de traitement de l'image, ainsi qu'avec
• une unité de commande pour la commande de l'unité de balayage, du dispositif d'entraînement et de l'unité de reconstruction,
dans lequel l'unité de commande est programmée de telle sorte que les étapes suivantes soient exécutées pour la production d'un scannogramme :
a) exécution d'un mouvement relatif entre l'unité de balayage et une zone d'examen qui comprend une rotation de l'unité de balayage autour de l'axe de rotation et une avance dans la direction parallèle à l'axe de rotation pendant l'acquisition des valeurs de mesure;
b) reconstruction de la distribution de l'absorption dans la zone d'examen comme un jeu de données en 3D composé de voxels, l'atténuation du rayonnement dans les différents voxels de la zone d'examen définis par leur position (x, y, z) étant calculée à partir des valeurs de mesure qui représentent l'intégrale de ligne de l'atténuation le long du faisceau respectif;
c) calcul d'au moins un scannogramme comme image de projection synthétique à partir du jeu de données en 3D reconstruit, le jeu de données en 3D reconstruit étant projeté sur une surface à partir d'une origine de projection à choisir librement.

5. Tomographe informatisé selon la revendication 4,
**caractérisé en ce**
**que** l'unité de commande est programmée de telle sorte que la rotation et l'avance soient effectuées simultanément pendant l'acquisition des valeurs de mesure de telle sorte qu'il en résulte un mouvement relatif hélicoïdal.

6. Tomographe informatisé selon la revendication 4,
**caractérisé en ce que** le faisceau de rayons émis par la source de rayons adopte la forme d'un cône et que l'unité à détecteurs comprenant le faisceau de rayons présente plusieurs lignes d'éléments détecteurs décalées l'une par rapport à l'autre parallèlement à l'axe de rotation.

7. Programme informatique pour une unité de commande pour la commande de l'unité de balayage, du dispositif d'entraînement et de l'unité de reconstruction d'un tomographe informatisé pour l'exécution du procédé selon la revendication 1, le programme informatique exécutant pour la production d'un scannogramme les étapes suivantes :
a) exécution d'un mouvement relatif entre l'unité de balayage et une zone d'examen qui comprend une rotation de l'unité de balayage autour de l'axe de rotation et une avance dans la direction parallèle à l'axe de rotation pendant l'acquisition des valeurs de mesure;
b) reconstruction de la distribution de l'absorption dans la zone d'examen comme un jeu de données en 3D composé de voxels, l'atténuation du rayonnement dans les différents voxels de la zone d'examen définis par leur position (x, y, z) étant calculée à partir des valeurs de mesure qui représentent l'intégrale de ligne de l'atténuation le long du faisceau respectif;
c) calcul d'au moins un scannogramme comme image de projection synthétique à partir du jeu de données en 3D reconstruit, le jeu de données en 3D reconstruit étant projeté sur une surface à partir d'une origine de projection à choisir librement.
